# EUROPÄISCHE PATENTANMELDUNG

(11) **EP 4 614 053 A1**
(43) Veröffentlichungstag der Anmeldung: **10.09.2025**
(21) Anmeldenummer: 25159184.8
(22) Anmeldetag: 20.02.2025
(51) Int. Cl.: F16M 13/02, F16M 11/08, A61B 90/50, F16M 11/20, F16M 11/22, F16L 21/06, F16B 21/18

(54) **ANORDNUNG ZUR AXIALSICHERUNG EINER ACHSE IN EINER ACHSAUFNAHME**

(30) Priorität: 08.03.2024 DE 102024106776
(71) Anmelder: Karl Leibinger Asset Management GmbH & Co. KG, 78570 Mühlheim an der Donau (DE)
(72) Erfinder: Strölin, Joachim, 78604 Rietheim-Weilheim (DE)
(74) Vertreter: Schwamberger, Martin

(57) **Zusammenfassung**

Anordnung (Z) geeignet zur Axialsicherung einer Achse (A) in einer Achsaufnahme (N), mit einer ersten Sicherungsscheibe (S1) und einer gegenüberliegend angeordneten zweiten Sicherungsscheibe (S2), wobei durch Verschwenken der Sicherungsscheiben (S1, S2) diese in eine Sicherungs-Position und eine Offen-Position bringbar sind, wobei die Sicherungsscheiben (S1, S2) je eine Ausnehmung (A1, A2) aufweisen, wobei jeder Sicherungsscheibe (S1, S2) ein Sicherungsstift (ST1, ST2) zugeordnet ist, wobei die Ausnehmungen (A1, A2) und die Sicherungsstifte (ST1, ST2) derart gestaltet sind, dass durch Verschwenken zumindest einer der beiden Sicherungsscheiben (S1, S2) von der Offen-Position in die Sicherungs-Position der jeweilige Sicherungsstift (ST1, ST2) in die jeweilige Ausnahme (A1, A2) einrastet und derart ein Zurückschwenken der Sicherungsscheibe (S1, S2) in die Offen-Position formschlüssig verhindert.

## Beschreibung

Die Erfindung betrifft eine Anordnung zur Axialsicherung einer Achse in einer Achsaufnahme. Die Erfindung betrifft ferner eine Achse und eine Achsaufnahme zur Aufnahme der Achse, ein medizinisches Gerät mit einer solchen Achse und Achsaufnahme, sowie ein Verfahren zur Montage einer Achse in einer Achsaufnahme.

Im Stand der Technik sind verschiedene Anordnungen zur Axialsicherung einer Achse in einer Achsaufnahme bekannt. So lehrt beispielsweise die EP 3 009 728 A1 ein Sicherungselement zum Sichern einer Spindel an einer Buchse. Das Sicherungselement ist plattenförmig ausgebildet und weist einen Eingriffsabschnitt auf, der in der Sicherungsposition mit einer Nut einer Spindel zusammenwirkt. Die Konstruktion ist für die Befestigung eines Tragarms einer medizintechnischen Einrichtung im Operationssaal bestimmt.

Bei einer derartigen Anwendung muss die Axialsicherung besonders robust gegen eine fehlerhafte Montage sein, da sich im Fehlerfall der Tragarm von der Befestigung lösen und im Operationssaal herabfallen könnte. Gleichzeitig sind derartige Systeme verhältnismäßig schwer, und werden üblicherweise per Hand von einem oder zwei Monteuren an der Decke des Operationssaals befestigt.

Die DE 20 2008 012 151 U1 beschreibt einen Klappring zur Anordnung auf einer Welle oder Walze. Der Klappring weist zwei Ringsegmente auf. An einem Ende der Ringsegmente sind diese an einem Bolzen drehbar festgelegt, sodass die Ringsegmente im Sinne eines Auf- bzw. Zuklappens verschwenkt werden können, und so in einer Nut der Walze eingreifen. Am anderen Ende der Ringsegmente sind diese über einen selbstsichernden Schnellverschluss miteinander verbindbar.

Eine derartige Lösung wäre für eine Deckenmontage nur bedingt geeignet. Ist die Lage der Nut beispielsweise nicht sichtbar, so könnte der Monteur versuchen den Schnellverschluss zu schließen, obwohl die Ringsegmente noch nicht vollständig in der Nut festsitzen.

Es ist also Aufgabe der Erfindung ein System zur Axialsicherung einer Achse in einer Achsaufnahme bereitzustellen, welches auch bei eingeschränkter Sichtbarkeit und hoher Last einfach und zuverlässig zu bedienen ist. Eine weitere Aufgabe der Erfindung ist es, ein entsprechendes Montageverfahren bereitzustellen.

Die Aufgabe wird gelöst durch die Merkmale des Patentanspruchs 1. Die weitere Aufgabe wird gelöst durch die Merkmale des Patentanspruchs 9. Vorteilhafte Ausgestaltungen ergeben sich aus den abhängigen Ansprüchen, der Beschreibung sowie aus den Figuren.

Zur Lösung der Aufgabe wird eine Anordnung zur Axialsicherung einer Achse in einer Achsaufnahme vorgeschlagen. Die Anordnung weist eine erste Sicherungsscheibe und eine gegenüberliegend angeordnete zweite Sicherungsscheibe auf. Die erste Sicherungsscheibe ist um eine erste Drehachse drehbar gelagert. Die zweite Sicherungsscheibe ist um eine zweite Drehachse drehbar gelagert. Durch Verschwenken um die jeweilige Drehachse sind die Sicherungsscheiben in eine Sicherungs-Position und eine Offen-Position bringbar. In der Sicherungs-Position liegt die Axialsicherung vor, während in der Offen-Position die Axialsicherung nicht vorliegt. Am radial äußeren Rand weisen die Sicherungsscheiben je eine Ausnehmung auf.

Erfindungsgemäß sind der ersten und zweiten Sicherungsscheibe je ein Sicherungsstift zugeordnet. Die Ausnehmungen und die Sicherungsstifte sind derart gestaltet, dass durch Verschwenken von zumindest einer der beiden Sicherungsscheiben ausgehend von der Offen-Position in die Sicherungs-Position der jeweilige Sicherungsstift in die jeweilige Ausnahme einrastet, und so ein Zurückschwenken der Sicherungsscheibe in die Offen-Position formschlüssig verhindert.

Eine derartige Lösung ist auch bei eingeschränkter Sichtbarkeit und hoher Last einfach und zuverlässig zu bedienen. Denn ein Monteur muss lediglich die Achse in die korrekte Lage relativ zur Achsaufnahme bringen und anschließend die Sicherungsscheiben durch einfaches Verschwenken in die Sicherungs-Position bringen. Da die Sicherungsstifte ein Zurückschwenken der Sicherungsscheiben in die Offen-Position verhindern, ist die Achse zumindest vorläufig axial gesichert. Der Montagevorgang kann nun weitergeführt werden.

Vorzugsweise sind die Sicherungsstifte räumlich in vertikaler Richtung angeordnet. Werden die Sicherungsscheiben von der Offen-Position in die Sicherungs-Position verschwenkt, so können die Sicherungsstifte bei einer derartigen Lösung selbsttätig durch Schwerkraft in die jeweilige Ausnehmung der Sicherungsscheiben einrasten. Ein Monteur müsste somit lediglich die Sicherungsscheiben in die Sicherungs-Position bringen.

Vorzugsweise ist das Einrasten der Sicherungsstifte in die Ausnehmungen von außen sichtbar, sodass der Monteur den gesicherten Zustand auf einfache Weise erkennt.

Gemäß einer alternativen Ausführung sind die Sicherungsstifte durch je eine Feder vorgespannt. Die Ausnehmungen der Sicherungsscheiben sind dabei derart gestaltet, dass der jeweilige Sicherungsstift beim Verschwenken der zugeordneten Sicherungsscheibe von der Offen-Position in die Sicherungs-Position aufgrund der Federvorspannung in die zugeordnete Ausnehmung einrastet. Beispielsweise könnten die Sicherungsstifte in je einer Aufnahme geführt sein, wobei eine Öffnung der Aufnahme durch die zugeordnete Sicherungsscheibe verdeckt wird, solange sich die Sicherungsscheibe in der Offen-Position befindet. Wird die Sicherungsscheibe in die Sicherungs-Position überführt, so gibt die Sicherungsscheibe die Öffnung der Aufnahme frei, sodass der Sicherungsstift aufgrund der Federvorspannung aus der Aufnahme hervortritt und in die Ausnehmung einrastet.

Vorzugsweise ist in der ersten und/oder zweiten Sicherungsscheibe je ein Durchgangsloch angeordnet. Das Durchgangsloch kann zur Aufnahme einer Schraube dienen, mittels der die Sicherungsscheibe in ihrer Sicherungs-Position festgelegt werden kann. Dadurch kann die Axialsicherung der Achse in der Achsaufnahme auf einfache Weise zusätzlich gesichert werden. Es ist auch denkbar, die Sicherungsstifte nach erfolgter Festlegung der Sicherungsscheiben in der Sicherungs-Position zu entfernen.

Gemäß einer möglichen Ausgestaltung sind die Durchgangslöcher derart dimensioniert, dass die Sicherungsstifte zumindest teilweise durch die Durchgangslöcher hindurchführbar sind. Die Sicherungsstifte sind bei einer solchen Ausgestaltung als Schrauben ausgebildet. Dadurch können die Sicherungsstifte entnommen, und zur endgültigen Festlegung der Sicherungsscheiben in der Sicherungs-Position verwendet werden.

Die erfindungsgemäß vorgeschlagene Anordnung kann Bestandteil eines Systems aus Achse und Achsaufnahme sein. Die Achse weist dazu an ihrem Außenumfang eine Ringnut auf. Die Sicherungsscheiben sind an der Achsaufnahme angeordnet und derart gestaltet, dass diese in der Sicherungs-Position in die Ringnut eingreifen und derart die Achse axial in der Achsaufnahme sichern.

Vorzugsweise ist ein Durchmesser der Achse in einem Bereich, welcher an die Ringnut anschließt, so groß, dass die Sicherungsscheiben nicht von der Offen-Position in die Sicherungs-Position schwenkbar sind, wenn die Position der Ringnut sich nicht mit den Sicherungsscheiben überdeckt. Dies führt zu einem besonders sicheren Montage-Prozess. Denn ein Verschwenken der Sicherungsscheiben in die Sicherungs-Position ist dann nur möglich, falls sich die Achse in der korrekten Lage befindet. Da aufgrund der Sicherungsstifte ein Zurückschwenken der Sicherungsscheiben in die Offen-Position auf einfache Weise verhindert wird, ermöglicht diese Ausgestaltung eine besonders zuverlässige axiale Sicherung.

Vorzugsweise kann das vorgeschlagene System aus Achse und Achsaufnahme Bestandteil eines medizinisches Geräts sein, beispielsweise eine chirurgische Leuchte zur Anwendung in einem Operationssaal. Das medizinische Gerät kann zumindest einen Federarm mit einer Achse aufweisen. An einem Ende der Achse kann der Federarm in einer Achsaufnahme aufgenommen werden. Achse und Achsaufnahme des medizinisches Geräts können dabei wie eingangs beschrieben gestaltet sein.

Zur Lösung der weiteren Aufgabe wird ein Verfahren zur Montage einer Achse in einer Achsaufnahme vorgeschlagen. Achse und Achsaufnahme sind dabei wie eingangs beschrieben ausgestaltet. Gemäß dem Verfahren wird die Achse zunächst durch eine Öffnung der Achsaufnahme hindurchgeführt, sodass Ringnut und Sicherungsscheiben in die gleiche axialen Position gelangen. Anschließend werden die ersten und zweiten Sicherungsscheiben von der Offen-Position in die Sicherungsposition verschwenkt, sodass die Sicherungsscheiben in die Ringnut einrasten und somit die Achse axial sichern.

Vorzugsweise werden, anschließend an das Verschwenken der Sicherungsscheiben in die Sicherungsposition, die Sicherungsscheiben in der Sicherungsposition festgelegt. Dies geschieht vorzugsweise durch festes Verschrauben der Sicherungsscheiben. Da die Sicherungsscheiben schwenkbar gelagert sind, führt eine weitere Verschraubung zu einer fest definierten Lage der Sicherungsscheiben. Dadurch wird eine besonders zuverlässige Axialsicherung erreicht.

Gemäß einer bevorzugten Ausgestaltung werden zur Festlegung der Sicherungsscheiben in der Sicherungsposition die Sicherungsstifte benutzt. Dazu wird zunächst der Sicherungsstift der ersten Sicherungsscheibe aus der jeweiligen Ausnehmung entnommen, und an einer anderen Stelle der ersten Sicherungsscheibe zur Festlegung derselben benutzt. Währenddessen verbleibt der Sicherungsstift der zweiten Sicherungsscheibe in der zugeordneten Ausnehmung, um die axiale Sicherungswirkung aufrechtzuerhalten. Ist die erste Sicherungsscheibe in ihrer Position festgelegt, wird der Sicherungsstift der zweiten Sicherungsscheibe aus der jeweiligen Ausnehmung entnommen und an einer anderen Stelle der zweiten Sicherungsscheibe zur Festlegung derselben benutzt.

Ausführungsbeispiele der Erfindung sind anhand der Figuren detailliert beschrieben. Es zeigen:
- Fig. 1-3: eine Achse und eine Achsaufnahme mit einer Anordnung zur Axialsicherung in verschiedenen Positionen,
- Fig. 4-5: eine alternative Anordnung zur Axialsicherung in verschiedenen Positionen sowie
- Fig. 6: ein medizinisches Gerät mit einer Achse und einer Achsaufnahme.

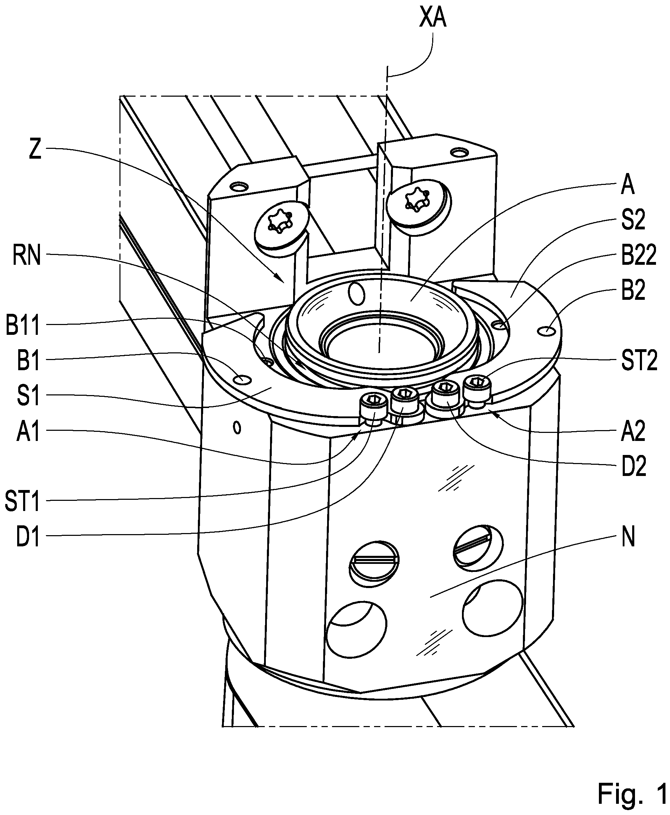

Fig. 1 zeigt eine Achse A und eine Achsaufnahme N zur Aufnahme der Achse A. Die Achse A ist in der Achsaufnahme N drehbar gelagert, sodass sich die Achse A um die Drehachse XA drehen kann. Die Drehachse XA ist räumlich vertikal ausgerichtet. Um zu vermeiden, dass die Achse A sich aus der Achsaufnahme N löst, ist eine Anordnung Z zur Axialsicherung der Achse A in der Achsaufnahme N vorgesehen.

Die Anordnung Z umfasst eine erste Sicherungsscheibe S1 und eine zweite Sicherungsscheibe S2. Die beiden Sicherungsscheiben S1, S2 sind an der Achsaufnahme N drehbar gelagert. Die Sicherungsscheibe S1 ist dazu an einem Ende mittels einer Schraube D1 an der Achsaufnahme N aufgenommen. Die Sicherungsscheibe S2 ist an einem Ende mittels einer Schraube D2 an der Achsaufnahme N aufgenommen. Die Sicherungsscheiben S1, S2 weisen die Form eines halben Kreissegments auf. Die Sicherungsscheiben S1, S2 weisen in unmittelbarer räumlicher Nähe zur ihrer drehbaren Aufnahme je eine Ausnehmung A1, A2 auf. Die Ausnehmungen A1, A2 sind am radial äußeren Rand der jeweiligen Sicherungsscheibe S1, S2 angeordnet. In der Darstellung gemäß Fig. 1 sind die Sicherungsscheiben S1, S2 in einer Offen-Position angeordnet. In dieser Offen-Position kann die Achse A entlang der Drehachse XA durch eine Öffnung der Achsaufnahme N hindurchgeführt werden. Im Bereich der Ausnehmungen A1, A2 ist je ein Sicherungsstift ST1, ST2 angeordnet. Die Sicherungsstifte ST1, ST2 sind als Schrauben ausgeführt und weisen einen Schraubenkopf auf. In der Offen-Position der Sicherungsscheiben S1, S2 liegt der Schraubenkopf der Sicherungsstifte ST1, ST2 auf der jeweiligen Sicherungsscheibe S1, S2 auf.

Die Achse A weist an ihrem Außendurchmesser eine Ringnut RN auf. Durch Verschwenken der Sicherungsscheiben S1, S2 in Richtung der Achse A rasten die Sicherungsscheiben S1, S2 in die Ringnut RN ein, sofern die Ringnut RN sich auf derselben axialen Position wie die Sicherungsscheiben S1, S2 befinden. Anschließend an die Ringnut RN ist ein Durchmesser der Achse A so groß, dass die Sicherungsscheiben S1, S2 nicht so weit in Richtung der Achse A geschwenkt werden können, dass der Schraubenkopf der Sicherungsstifte ST1, ST2 nicht mehr auf der jeweiligen Sicherungsscheibe S1, S2 aufliegt. Erst wenn sich die Ringnut RN auf der Höhe der Sicherungsscheiben S1, S2 befindet, können die Sicherungsscheiben S1, S2 in die Ringnut RN einrasten, wodurch die Position der Achse A in Richtung der Drehachse XA relativ zur Achsaufnahme N festgelegt ist.

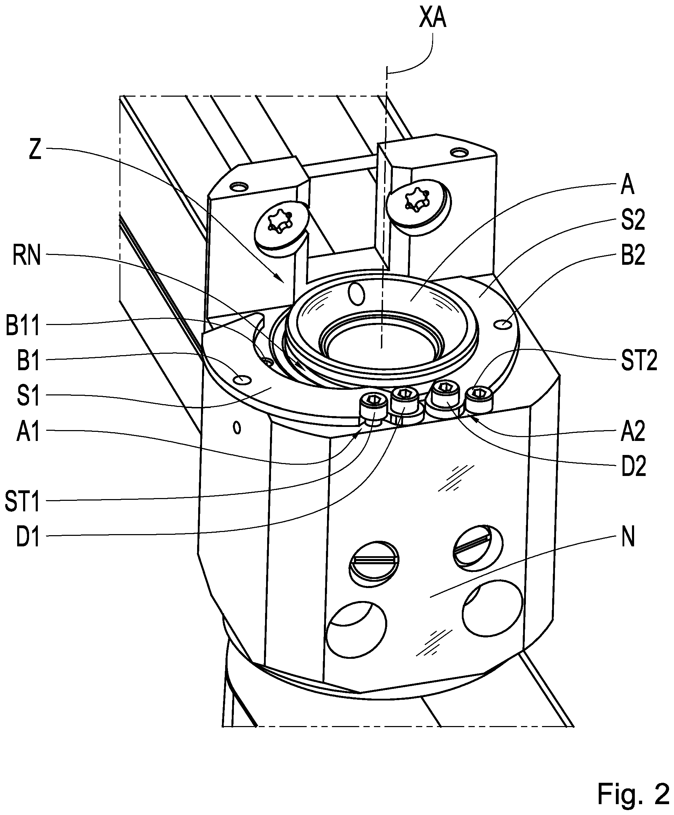

In Fig. 2 ist ein Zustand dargestellt, in dem die Sicherungsscheibe S2 von der Offen-Position in eine Sicherungs-Position verschwenkt wurde. Durch das Verschwenken der Sicherungsscheibe S2 in die Sicherungs-Position kann der Schraubenkopf des Sicherungsstifts ST2 nicht mehr auf der Sicherungsscheibe S2 aufliegen, und rutscht schwerkraftgetrieben nach unten. Dadurch rastet der Schraubenkopf des Sicherungsstifts ST2 in die Ausnehmung A2 der Sicherungsscheibe S2 ein, und verhindert somit formschlüssig ein Zurückschwenken der Sicherungsscheibe S2 in die Offen-Position. In der Sicherungsposition überdeckt sich ein Durchgangsloch B2 in der Sicherungsscheibe S2 mit einer Gewindebohrung B22 in der Achsaufnahme N, sodass eine Schraube durch das Durchgangsloch B2 in die Gewindebohrung B22 eingeführt werden kann. Auf diese Weise kann die Sicherungsscheibe S2 in ihrer Sicherungs-Position fest fixiert werden. Die Sicherungsscheibe S1 weist ebenso ein Durchgangsloch B1 auf. Befindet sich die Sicherungsscheibe S1 in der Sicherungs-Position, so überdeckt sich das Durchgangsloch B1 mit einer Gewindebohrung B11 der Achsaufnahme N, sodass eine Schraube durch das Durchgangsloch B1 in die Gewindebohrung B11 eingeführt werden kann. Auf diese Weise kann auch die Sicherungsscheibe S1 in ihrer Sicherungs-Position fest fixiert werden.

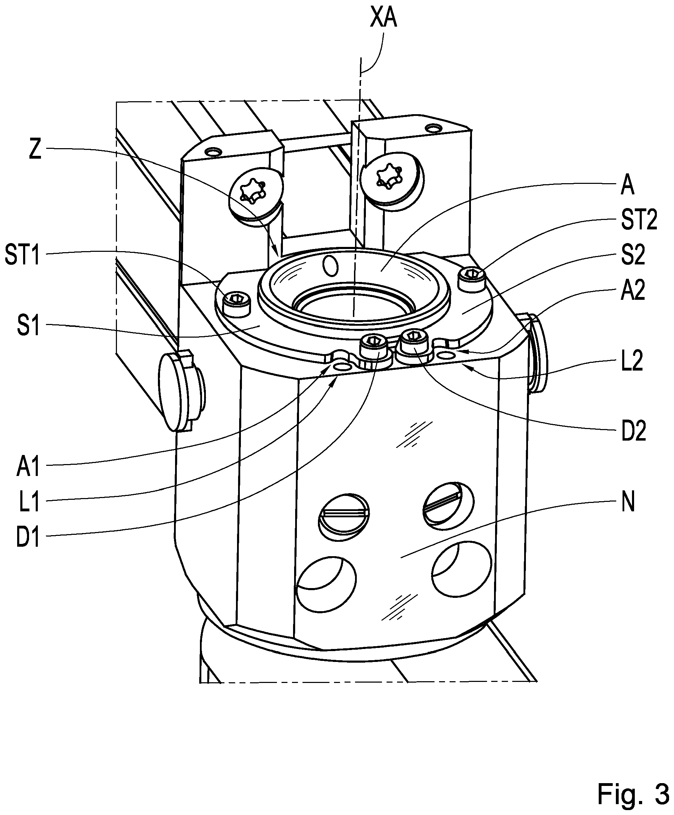

Fig. 3 zeigt einen Zustand, in dem beide Sicherungsscheiben S1, S2 in die Sicherungs-Position verschwenkt wurden. Die Sicherungsstifte ST1, ST2 wurden dabei aus ihren jeweiligen Aufnahmen L1, L2 herausgenommen, in die Durchgangslöcher B1, B2 der Sicherungsscheiben S1, S2 eingesetzt, und in die Gewindebohrungen B11, B22 der Achsaufnahme N verschraubt. Alternativ dazu könnten die Sicherungsstifte ST1, ST2 in ihren jeweiligen Aufnahmen L1, L2 verbleiben, und es könnten separate Schrauben zur Fixierung der Sicherungsscheiben S1, S2 in die Durchgangslöcher B1, B2 eingeführt werden. Aus Gründen der Übersichtlichkeit ist diese Variante in den Figuren nicht separat dargestellt.

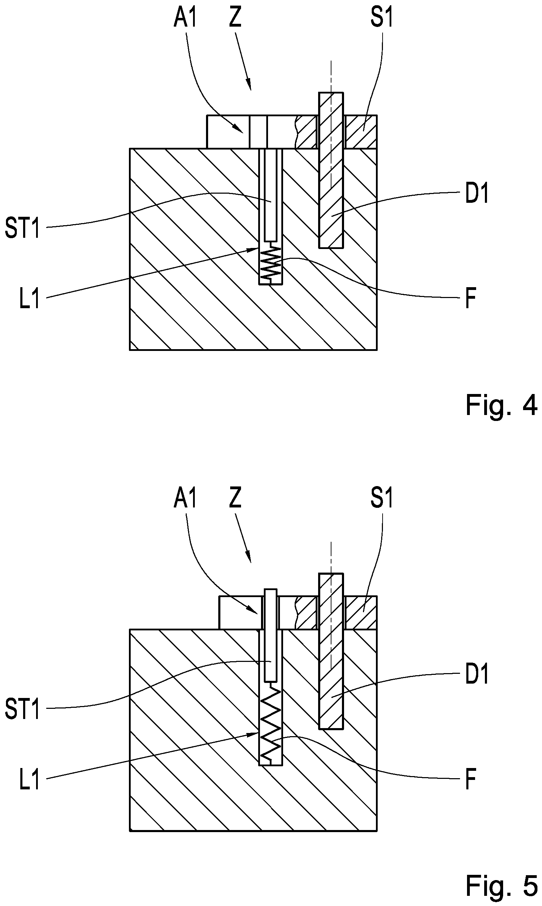

Fig. 4 und Fig. 5 zeigen eine schematische Darstellung gemäß einer alternativen Ausführung der Anordnung Z zur Axialsicherung. Zur besseren Übersichtlichkeit ist nur eine Hälfte der Anordnung Z dargestellt, also nur die Sicherungsscheibe S1 mit ihrer Ausnehmung A1 sowie der Sicherungsstift ST1 in seiner Ausnehmung L1. Selbstverständlich weist auch die alternative Ausführung der Anordnung Z gemäß Fig. 4 und Fig. 5 zwei Sicherungsscheiben S1, S2 sowie zwei Sicherungsstifte ST1, ST2 auf.

In der Darstellung gemäß Fig. 4 befindet sich die Sicherungsscheibe S1 in der Offen-Position. Der Sicherungsstift ST1 ist mittels einer Feder F vorgespannt. Das der Sicherungsscheibe S1 zugewandte Ende des Sicherungsstifts ST1 liegt dabei an einer Oberfläche der Sicherungsscheibe S1 an, da die Ausnehmung A1 der Sicherungsscheibe S1 sich nicht mit der Aufnahme L1 überdeckt. In der Darstellung gemäß Fig. 5 befindet sich die Sicherungsscheibe S1 in der Sicherungs-Position. In der Sicherungs-Position überdeckt sich die Ausnehmung A1 mit der Aufnahme L1, sodass der Sicherungsstift ST1 getrieben durch die Feder F teilweise aus der Aufnahme L1 herausgedrückt wird, und in die Ausnehmung A1 einrastet. Dadurch kann die Sicherungsscheibe S1 nicht mehr in die Offen-Position zurückschwenken.

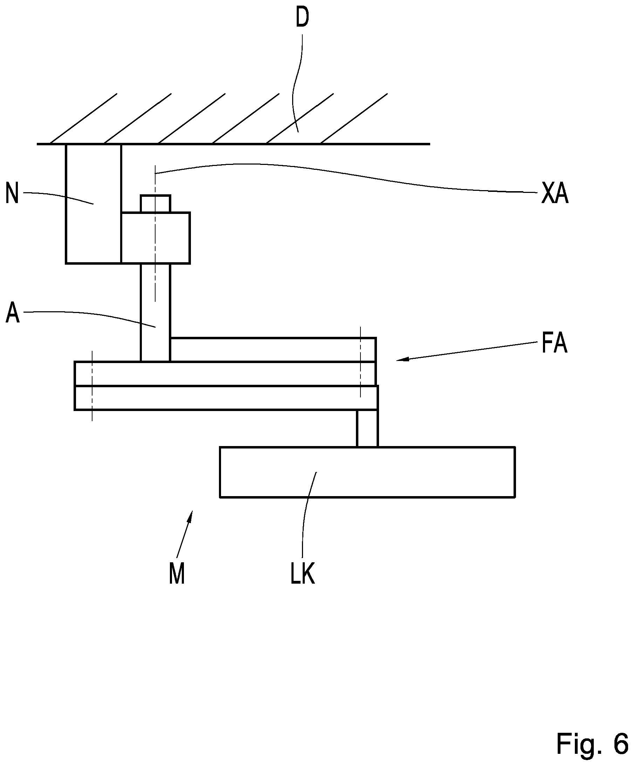

Fig. 6 zeigt ein medizinisches Gerät M, insbesondere eine Leuchte zur Anwendung in einem Operationssaal. Das medizinische Gerät M weist einen Federarm FA zur räumlichen Positionierung eines Leuchtkörpers LK auf. Der Federarm FA weist eine Achse A auf, wobei ein Ende der Achse A in einer Achsaufnahme N aufgenommen ist. Die Achsaufnahme N ist an einer Zimmerdecke D fest fixiert. Achse A und Achsaufnahme N können entsprechend eines der oben beschriebenen Ausführungsbeispiele ausgeführt sein.

Bei der Montage des medizinischen Geräts M wird zunächst die Achsaufnahme N an der Zimmerdecke D montiert. Anschließend wird die Achse A in die Achsaufnahme N eingeführt, und mittels in der Anordnung Z zur Axialsicherung fixiert, in dem die beiden Sicherungsscheiben S1, S2 von der Offen-Position in die Sicherungs-Position verschwenkt werden. Anschließend werden die Sicherungsscheiben S1, S2 in ihrer Sicherungs-Position festgelegt.

### Bezugszeichenliste

- A: Achse
- RN: Ringnut
- N: Achsaufnahme
- XA: Drehachse
- Z: Anordnung
- S1: Erste Sicherungsscheibe
- B1: Durchgangsloch
- B11: Gewindebohrung
- S2: Zweite Sicherungsscheibe
- B2: Durchgangsloch
- B22: Gewindebohrung
- D1: Schraube
- D2: Schraube
- A1: Ausnehmung
- A2: Ausnehmung
- ST1: Sicherungsstift
- ST2: Sicherungsstift
- L1: Aufnahme
- L2: Aufnahme
- F: Feder
- M: Medizinisches Gerät
- FA: Federarm
- LK: Arbeitsende
- D: Zimmerdecke

## Patentansprüche

1. Anordnung (Z) zur Axialsicherung einer Achse (A) in einer Achsaufnahme (N), mit einer ersten Sicherungsscheibe (S1) und einer gegenüberliegend angeordneten zweiten Sicherungsscheibe (S2),
wobei die erste Sicherungsscheibe (S1) um eine erste Drehachse drehbar gelagert ist und die zweite Sicherungsscheibe (S2) um eine zweite Drehachse drehbar gelagert ist, sodass die erste und die zweite Sicherungsscheibe (S1, S2) durch Verschwenken um die jeweilige Drehachse in eine Sicherungs-Position und eine Offen-Position bringbar sind, wobei die erste und zweite Sicherungsscheibe (S1, S2) am radial äußeren Rand je eine Ausnehmung (A1, A2) aufweisen,
**dadurch gekennzeichnet, dass** der ersten und zweiten Sicherungsscheibe (S1, S2) je ein Sicherungsstift (ST1, ST2) zugeordnet ist, wobei die Ausnehmungen (A1, A2) und die Sicherungsstifte (ST1, ST2) derart gestaltet sind, dass durch Verschwenken zumindest einer der beiden Sicherungsscheiben (S1, S2) von der Offen-Position in die Sicherungs-Position der jeweilige Sicherungsstift (ST1, ST2) in die jeweilige Ausnahme (A1, A2) einrastet und derart ein Zurückschwenken der Sicherungsscheibe (S1, S2) in die Offen-Position formschlüssig verhindert.

2. Anordnung (Z) nach Anspruch 1, **dadurch gekennzeichnet, dass** die Sicherungsstifte (ST1, ST2) räumlich in vertikaler Richtung angeordnet sind, sodass das Einrasten des jeweiligen Sicherungsstifts (ST1, ST2) in die dazugehörige Ausnehmung (A1, A2) beim Verschwenken der jeweiligen Sicherungsscheibe (S1, S2) von der Offen-Position in die Sicherungs-Position selbsttätig durch Schwerkraft erfolgt.

3. Anordnung (Z) nach Anspruch 1, **dadurch gekennzeichnet, dass** die Sicherungsstifte (ST1, ST2) durch je eine Feder (F) vorgespannt sind, wobei die Ausnehmungen (A1, A2) derart gestaltet sind, dass beim Verschwenken der jeweiligen Sicherungsscheibe (S1, S2) von der Offen-Position in die Sicherungs-Position der jeweilige Sicherungsstift (ST1, ST2) aufgrund der Federvorspannung in die jeweilige Ausnahme (A1, A2) einrastet.

4. Anordnung (Z) nach einem der Ansprüche 1 bis 3, **dadurch gekennzeichnet, dass** in der ersten und/oder zweiten Sicherungsscheibe (S1, S2) je ein Durchgangsloch (B1, B2) angeordnet ist.

5. Anordnung (Z) nach Anspruch 4, **dadurch gekennzeichnet, dass** die Durchgangslöcher (B1, B2) derart gestaltet sind, dass die Sicherungsstifte (ST1, ST2) durch die Durchgangslöcher (B1, B2) zumindest teilweise hindurchführbar sind, wobei die Sicherungsstifte (ST1, ST2) als Schrauben ausgebildet sind.

6. Achse (A) und Achsaufnahme (N) zur Aufnahme der Achse (A), wobei die Achse (A) an ihrem Außenumfang eine Ringnut (RN) aufweist, **dadurch gekennzeichnet, dass** die Achsaufnahme (N) eine Anordnung (Z) zur Axialsicherung der Achse (A) nach einem der Ansprüche 1 bis 5 aufweist, wobei die Sicherungsscheiben (S1, S2) an der Achsaufnahme (N) angeordnet und derart gestaltet sind, dass diese in der Sicherungs-Position in die Ringnut (RN) eingreifen und so die Achse (A) axial in der Achsaufnahme (N) sichern.

7. Achse (A) und Achsaufnahme (N) nach Anspruch 6, **dadurch gekennzeichnet, dass** ein Durchmesser der Achse (A) im Bereich anschließend an die Ringnut (RN) so groß ist, dass die Sicherungsscheiben (S1, S2) nicht von der Offen-Position in die Sicherungs-Position schwenkbar sind, wenn die Position der Ringnut (RN) sich nicht mit den Sicherungsscheiben (S1, S2) überdeckt.

8. Medizinisches Gerät (M), insbesondere chirurgische Leuchte, mit zumindest einem Federarm (FA) zur räumlichen Positionierung eines Arbeitsendes (LK) des medizinischen Geräts (M), wobei der zumindest eine Federarm (FA) eine Achse (A) aufweist, wobei ein Ende der Achse (A) in einer fest fixierten Achsaufnahme (N) aufgenommen ist, **dadurch gekennzeichnet, dass** die Achse (A) und die Achsaufnahme (N) gemäß Anspruch 6 oder Anspruch 7 ausgebildet sind.

9. Verfahren zur Montage einer Achse (A) in einer Achsaufnahme (N) gemäß Anspruch 6 oder Anspruch 7, **gekennzeichnet durch** folgende Schritte:
a) Durchführen der Achse (A) durch eine Öffnung der Achsaufnahme (N),
b) Verschwenken der ersten und zweiten Sicherungsscheibe (S1, S2) von der Offen-Position in die Sicherungsposition.

10. Verfahren nach Anspruch 9, **dadurch gekennzeichnet, dass**, nach dem Verschwenken der ersten und zweiten Sicherungsscheibe (S1, S2) von der Offen-Position in die Sicherungs-Position, die Sicherungsscheiben (S1, S2) in der Sicherungs-Position festgelegt werden.

11. Verfahren nach Anspruch 10, **dadurch gekennzeichnet, dass** zur Festlegung der Sicherungsscheiben (S1, S2) in der Sicherungsposition die Sicherungsstifte (ST1, ST2) benutzt werden, wobei
zunächst der Sicherungsstift (ST1) der ersten Sicherungsscheibe (S1) aus der jeweiligen Ausnehmung (A1) entnommen wird und an einer anderen Stelle der ersten Sicherungsscheibe (S1) zur Festlegung derselben benutzt wird, während der Sicherungsstift (ST2) der zweiten Sicherungsscheibe (S2) in der jeweils anderen Ausnehmung (A2) verbleibt,
und anschließend daran der Sicherungsstift (ST2) der zweiten Sicherungsscheibe (S2) aus der jeweiligen Ausnehmung (A2) entnommen wird und an einer anderen Stelle der zweiten Sicherungsscheibe (S2) zur Festlegung derselben benutzt wird.
